(19) Europäisches Patentamt

European Patent Office

Office européen des brevets

(11) Publication number: **0 323 268 B1**

(12) **EUROPEAN PATENT SPECIFICATION**

(45) Date of publication of patent specification: **10.03.93**

(51) Int. Cl.5: **C07C 29/04**, C07C 31/10, C07C 41/06, C07C 43/04, C07C 31/12

(21) Application number: **88312425.7**

(22) Date of filing: **30.12.88**

(54) **Process for the catalytic hydration of olefins.**

(30) Priority: **30.12.87 US 139570**

(43) Date of publication of application:
**05.07.89 Bulletin 89/27**

(45) Publication of the grant of the patent:
**10.03.93 Bulletin 93/10**

(84) Designated Contracting States:
**BE DE FR GB IT NL SE**

(56) References cited:
**EP-A- 0 055 045**
**EP-A- 0 186 395**
**EP-A- 0 213 884**
**EP-A- 0 323 137**
**EP-A- 0 323 270**

(73) Proprietor: **MOBIL OIL CORPORATION**
**3225 Gallows Road**
**Fairfax, Virginia 22037-0001(US)**

(72) Inventor: **Bell, Weldon K.**
**428 Burd Street**
**Pennington, NJ 08534(US)**
Inventor: **Haag, Werner O.**
**38 Pine Knoll Drive**
**Lawrenceville, NJ 08648(US)**
Inventor: **Huang, Tracy J.**
**9 Woodfield Lane**
**Lawrenceville, NJ 08648(US)**
Inventor: **Varghese, Philip**
**8 Chadwick Dr.**
**Voorhees, NJ 08043(US)**

(74) Representative: **Jones, Alan John et al**
**CARPMAELS & RANSFORD 43 Bloomsbury Square**
**London, WC1A 2RA (GB)**

Rank Xerox (UK) Business Services
(3.10/3.5x/3.0.1)

**EP 0 323 268 B1**

**Description**

This invention relates to a process for the catalytic hydration of olefins to provide alcohols, ethers and their mixtures. More particularly, the invention relates to a process for the hydration of light olefins such as ethylene, propylene, butenes, pentenes, hexenes, heptenes, etc., and their mixtures to provide a mixture of alcohol(s) and ether(s) employing acidic zeolite Beta as catalyst. The co-produced alcohols and ethers are useful, inter alia, as high octane blending stocks for gasolines.

There is a need for an efficient catalytic process to manufacture alcohols and ethers from light olefins thereby augmenting the supply of high octane blending stocks for gasoline. Lower molecular weight alcohols and ethers such as isopropyl alcohol (IPA) and diisopropyl ether (DIPE) are in the gasoline boiling range and are known to have a high blending octane number. In addition, by-product propylene from which IPA and DIPE can be made is usually available in a fuels refinery. The petrochemicals industry also produces mixtures of light olefin streams in the $C_2$ to $C_7$ molecular weight range and the conversion of such streams or fractions thereof to alcohols and/or ethers can also provide products useful as solvents and as blending stocks for gasoline.

The catalytic hydration of olefins to provide alcohols and ethers is a well-established art and is of significant commercial importance. US-A-4,214,107 discloses catalytic hydration of propylene over a crystalline aluminosilicate zeolite catalyst having a silica to alumina ratio of at least 12 and a Constraint Index of from 1 to 12, e.g. HZSM-5 type zeolite, to provide the corresponding alcohol, essentially free of ether and hydrocarbon by-product.

According to US-A-4,499,313 an olefin is hydrated to the corresponding alcohol in the presence of H-mordenite or H-Y each having a silica-alumina molar ratio of 20 to 500. The use of such a catalyst is said to result in higher yields of alcohol than olefin hydration processes which employ conventional solid acid catalysts. Use of the catalyst is said to offer the advantage over ion-exchange type olefin hydration catalysts of not being restricted by the hydration temperature. Reaction conditions employed in the process include a temperature of from 50-300ºC, preferably 100-250ºC, a pressure of 5 to 200 kg/cm$^2$ to maintain liquid phase or gas-liquid multiphase conditions and a mole ratio of water to olefin of from 1 to 20. The reaction time can be 20 minutes to 20 hours when operating batchwise and the liquid hourly space velocity (LHSV) is usually 0.1 to 10 in the case of continuous operation.

EP-A-323270 describes olefin hydration over zeolite both at water/olefin mole ratios less than 1.

EP-A-210,793 describes an olefin hydration process employing a medium pore zeolite as hydration catalyst. Specific catalysts mentioned are Theta-1, said to be preferred, ferrierite, ZSM-22, ZSM-23 and NU-10.

According to the present invention a process for converting light olefin(s) to alcohol(s), ether(s) or a mixture of alcohol(s) and ether(s) is provided which comprises contacting a feed containing at least one light olefin with water in the vapour and/or liquid phase at a water to olefin mole ration of at least 1 under olefin hydration conditions in the presence of zeolite Beta as catalyst to produce alcohol(s), ether(s) or mixture thereof.

The alcohol(s), ether(s) and alcohol/ether mixtures resulting from the foregoing olefin hydration process are advantageously employed as blending components for gasoline, as cosolvents for methanol to be incorporated into gasoline, and many other applications.

The zeolite is preferably employed in the hydrogen form, and the catalyst may further comprise a co-catalyst effective for light olefin hydration. The light olefins particularly contemplated are those of 2 to 7 carbon atoms, including ethylene, propylene, butenes, pentenes, hexenes, heptenes and mixtures of these with each other or with other olefins.

Accordingly, the invention is applicable to the hydration of olefins contained in refinery streams such as gas plant off-gas containing ethylene and propylene, naphtha cracker off-gas containing light olefins, fluidized catalytic cracked (FCC) light gasoline containing pentenes, hexenes, and heptenes, refinery FCC propane/propylene streams. For example, a typical FCC light olefin stream possesses the following composition:

2

| Typical Refinery FCC Light Olefin Composition | | |
|---|---|---|
| | Wt.% | Mole% |
| Ethane | 3.3 | 5.1 |
| Ethylene | 0.7 | 1.2 |
| Propane | 14.5 | 15.3 |
| Propylene | 42.5 | 46.8 |
| Isobutane | 12.9 | 10.3 |
| n-Butane | 3.3 | 2.6 |
| Butenes | 22.1 | 18.3 |
| Pentanes | 0.7 | 0.4 |

The process of the invention is especially applicable to the conversion of propylene and propylene-containing streams to mixtures of IPA and DIPE, which may be employed as an octane improver for gasoline.

The operating conditions of the olefin hydration process herein are not especially critical. They include a temperature ranging from ambient up to about 300°C, preferably from about 50 to about 220°C and more preferably from about 90 to about 200°C, a total system pressure of at least about 5 atm, preferably at least about 20 atm and more preferably at least about 40 atm, a water to total olefin mole ratio of up to about 30, preferably up to about 15 and most preferably up to about 5. Those skilled in the art will recognize that selection of specific operating conditions for a particular feed will influence product distribution. It will also be appreciated that the precise conditions selected should, to some extent, reflect the nature of the olefin feed, isoolefins generally requiring milder process conditions than straight chain olefins.

The olefin hydration process of this invention can be carried out under liquid phase, vapour phase or mixed vapour-liquid phase conditions in batch or continuous manner using a stirred tank reactor or fixed bed flow reactor, e.g., trickle-bed, liquid-up-flow, liquid-down-flow, counter-current, co-current, etc. Reaction times of from about 20 minutes to about 20 hours when operating in batch and an LHSV of from about 0.1 to about 10 when operating continuously are suitable. It is generally preferable to recover any unreacted olefin and recycle it to the reactor.

When seeking to maximise the production of ether by the hydration of olefin, the aqueous product effluent from the olefin hydration reactor containing both alcohol and ether olefin hydration products can be introduced into a separator, e.g., a distillation column, for recovery of ether. The dilute aqueous solution of alcohol may be then introduced into a second separator, e.g., another distillation column, where a water/alcohol azeotrope is recovered. A fraction of the azeotrope may be fed into a dehydration reactor of conventional or otherwise known type and operation to provide a further quantity of ether which can be combined with the ether previously recovered from the olefin hydration reactor. By blending various product streams, almost any ratio of alcohol/ether can be obtained. When alcohol/ether mixtures are to be used as gasoline blending stocks, this capability for adjusting the ratios of alcohol to ether offers great flexibility in meeting the octane requirements for given gasoline compositions. Regulatory considerations aside, alcohol/ether mixtures, e.g., IPA/DIPE mixtures, can constitute up to about 20 weight percent or so of the gasoline to which they are added.

A particularly advantageous procedure for producing mixtures of alcohol and ether, and in particular IPA and DIPE, from the hydration of an olefin-containing feed (a propylene-containing feed in the case of IPA/DIPE mixtures) comprises co-feeding a fresh propane/propylene-containing feed (readily available in many petroleum refineries) and fresh water, together with recycled unreacted propylene and recycled water from a decanter, into a hydration reactor. The reactor effluent is passed to a separator unit with propane and unconverted propylene being recycled to the reactor, part of the gaseous mixture being purged in order to avoid build-up of propane in the recycle loop. The liquid products from the separator unit are introduced to a distillation unit where an azeotropic mixture of IPA, DIPE, water and propylene oligomers (mostly $C_6$ olefin) is distilled off and, following cooling, is introduced into a decanter in which phase separation takes place. The upper layer contains mostly DIPE, e.g., 90 weight percent or more, and relatively little water, e.g., 1 weight percent or so. The lower layer is largely water containing negligible quantities of IPA and DIPE. The quantity of the decanter overhead which is recycled can be regulated so as to control the water content in the final product. The bottom fraction from the distillation unit, mainly IPA, is combined with DIPE in the decanter overhead to provide the final IPA/DIPE mixture.

Where it is desired to separate out the alcohol from an alcohol/ether mixture and thus provide essentially pure ether, one can advantageously pass the effluent from the hydration reactor to a separator

3

operating below the olefin hydration reaction temperature, where two liquid phases form, the aqueous phase being removed and recycled to the hydration reactor. The hydrocarbon-rich phase is flashed to a lower pressure to effect separation of the unreacted $C_3$ components. The flashed product, now containing a substantial amount of IPA product, is introduced to a distillation unit operated at or below atmospheric pressure to effect further purification of the DIPE. The azeotropic IPA, DIPE and water overhead product containing a small amount of propylene oligomer is condensed and thereafter contacted with reactor feed water. The resulting phase separation provides a DIPE product containing at most negligible amounts of IPA and water, e.g., 1.0 weight percent and 0.5 weight percent of these materials, respectively. The remaining aqueous phase can be recycled to the reactor.

The catalyst employed in the olefin hydration process of this invention, acidic zeolite Beta, is a large pore aluminosilicate zeolite which, like other large pore zeolites, possesses a Constraint Index of no greater than about 2 but in several respects does not behave like other large pore zeolites. Zeolite Beta is described in US-A-3,308,069, to which reference is made for further details. In general, the zeolite employed herein will possess a silica to alumina ratio of greater than about 10 and usually greater than about 20. In addition to aluminium present in the framework structure of zeolite Beta, other metals can be present such as gallium, iron, boron, etc.

The zeolite Beta catalyst used herein will generally possess an alpha value of at least about 1, preferably at least about 10 and more preferably at least about 100. (The alpha test is described in J. Catalysis, 6, pp. 278-287 (1966).) Zeolite Beta of low acidity (alpha values of less than about 200) can be achieved by a variety of techniques including (a) synthesising the zeolite with a high silica/alumina ratio, (b) steaming, (c) steaming followed by dealuminsation and (d) substituting aluminium with one or more other species. For example, in the case of steaming, the zeolite can be exposed to steam at elevated temperatures ranging from about 500 to about 1200°F (260 to 649°C) and preferably from about 750 to about 1000°F (339 to 538°C). This treatment can be accomplished in an atmosphere of 100% steam or an atmosphere consisting of steam and a gas which is substantially inert to the zeolite. A similar treatment can be accomplished at lower temperatures employing elevated pressure, e.g., at form about 350 to about 700°F (177 to 371°C) with from about 10 to 200 atmospheres (10.14 to 202.76 bar). Specific details of several steaming procedures may be gained from the disclosures of US-A-4,325,994, 4,347,296, and 4,418,235. Aside from or in addition to any of the foregoing procedures, the surface acidity of the zeolite can be eliminated or reduced by treatment with bulky reagents as described in US-A-4,520,221.

Zeolite Beta catalysts having an alpha of from about 100 to about 600 may be especially preferred for converting propylene feeds.

In practising the olefin hydration process of the present invention, it can be advantageous to composite the zeolite Beta within a matrix, or binder, material which is resistance to the temperature and other conditions employed in the process. Useful matrix materials include both synthetic and naturally-occurring substances, e.g., inorganic materials such as clay, silica and/or metal oxides such as alumina. The latter can be either naturally-occurring or can be provided in the form of gelatinous precipitates or gels including mixtures of silica and metal oxides. Naturally-occurring clays which can be composited with the zeolite include those of the montmorillonite and kaolin families, which families include the sub-bentonites and the kaolins commonly known as Dixie, McNamee-Georgia and Florida clays or others in which the main mineral constituent is haloysite, kaolinite, dickite, nacrite or anauxite. Such clays can be used in the raw state as originally mined or initially subjected to calcination, acid treatment or chemical modification.

In addition to the foregoing materials, zeolite Beta can be composited with a porous matrix material such as carbon, alumina, silica, titania, zirconia, silica-alumina, silica-magnesia, silica-zirconia, silica-thoria, silica-beryllia and silica-titania, etc, as well as ternary oxide compositions such as silica-alumina-thoria, silica-alumina-zirconia, silica-alumina-magnesia and silica-magnesia-zirconia. The matrix can be in the form of a cogel. The relative proportions of zeolite Beta and matrix material, on an anhydrous basis, can vary widely with the zeolite content ranging from between about 1 to about 99 wt%, and more usually in the range of about 5 to about 90 wt%, of the dry composite.

In some cases, it may be advantageous to provide the zeolite Beta hydration catalyst as an extrudate bound with a low acidity refractory oxide binder. In a preferred preparative technique a homogeneous mixture of zeolite Beta, water and a low acidity refractory oxide binder, e.g., silica, which contains at least an extrusion-facilitating amount of the binder in a colloidal state and which is substantially free of added alkali metal base and/or base salt, is formed into an extrudable mass. The mass is extruded and the resulting extrudate is dried and calcined.

The original cations associated with zeolite Beta utilized herein can be replaced by a wide variety of other cations according to techniques well known in the art, e.g., by ion-exchange. Typical replacing cations include hydrogen, ammonium, alkyl ammonium and metal cations, and their mixtures. Metal cations can also be introduced into the zeolite. In the case of replacing metallic cations, particular preference is given to metals of Groups IB to VIII of the Periodic Table, including, by way of example, iron, nickel, cobalt, copper, zinc, palladium, calcium, chromium, tungsten, molybdenum, rare earth metals. These metals can also be present in the form of their oxides.

The following examples are illustrative of the olefin hydration process of the present invention.

EXAMPLE 1

Zeolite Beta (hydrogen form; binder free) was employed in a number of hydration runs carried out at water:olefin mole ratios both at and below those disclosed for other large pore zeolites.

The conditions of the hydration runs and the results thereof are set forth in Table 1 as follows:

Table 1:  Propylene Hydration Under Varying Conditions

| Hydration Conditions | RUN 1 | 2 | 3 |
|---|---|---|---|
| Temp., °C | 200 | 202 | 202 |
| Pressure, psi (bar) | 255 | 255 | 255 |
| Water:Propylene Mole Ratio | 4.74 | 1.00 | 1.17 |
| LHSV, total cat. | 4.26 | 2.51 | 0.35 |
| WHSV, total cat. | 5.956 | 2.783 | 0.393 |
| CONVERSION, % | | | |
| Propylene | 10.37 | 9.13 | 9.06 |
| Water | 2.58 | 8.78 | 11.08 |
| IPA | 10.1 | 7.5 | 8.3 |
| DIPE | 0.1 | 0.0 | 0.2 |
| Hydrocarbon (HC) (propylene oligomer) | 0.1 | 1.7 | 0.6 |
| IPA g/hr/l cat. | 179.5 | 122.0 | 20.5 |
| HC g/hr/l cat. | 1.5 | 18.9 | 1.0 |
| DIPE g/hr/l cat. | 2.0 | 0.1 | 0.4 |
| IPA/IPA+HC+DIPE | 0.981 | 0.865 | 0.935 |

As these data show, by operating at a high space velocity at near equilibrium conversion, hydrocarbon coproduction is minimised at about 5%.

EXAMPLE 2

This example illustrates the effect of the feed water:propylene mole ratio on the coproduction of hydrocarbon employing zeolite Beta (bound with 35 weight percent alumina). The hydration conditions and results are set forth in Table 2, Runs 1,4,5 and 8 are comparative,

Table 2:  Effect of Water:Propylene Mole Ratio

| Hydration Conditions | RUN 1 | 2 | 3 | 4 | 5 | 6 | 7 | 8 |
|---|---|---|---|---|---|---|---|---|
| Temp., °C | 162 | 161 | 161 | 162 | 162 | 162 | 205 | 214 |
| Pressure, psi (bar) | 1000 (70) | 1000 | 1000 | 1000 | 1000 | 1000 | 255 (17.6) | 255 |
| Water:Propylene Mole Ratio | 0.48 | 2.28 | 3.57 | 0.74 | 0.57 | 1.76 | 1.00 | 0.98 |
| LHSV, total cat. | 0.48 | 0.66 | 0.35 | 0.13 | 0.49 | 0.61 | 14.35 | 19.77 |
| WHSV, total cat. | 0.495 | 0.823 | 0.469 | 0.141 | 0.514 | 0.728 | 13.601 | 21.822 |
| CONVERSION, % | | | | | | | | |
| Propylene | 30.91 | 31.41 | 60.30 | 62.63 | 25.52 | 24.52 | 23.39 | 4.79 |
| Water | 62.19 | 11.36 | 14.48 | 54.21 | 33.67 | 12.10 | 0.00 | 2.56 |
| IPA | 11.8 | 17.8 | 31.1 | 21.9 | 11.8 | 17.6 | 0.0 | 2.2 |
| DIPE | 17.6 | 12.4 | 24.1 | 36.7 | 12.8 | 6.9 | 0.0 | 0.0 |
| Hydrocarbon (HC) | 1.5 | 1.2 | 5.0 | 4.0 | 1.0 | 0.0 | 23.4 | 2.6 |
| IPA g/hr/l cat. | 38.3 | 56.3 | 44.4 | 17.2 | 36.6 | 55.7 | 0.0 | 277.9 |
| HC g/hr/l cat. | 3.4 | 2.7 | 5.0 | 2.2 | 2.1 | 0.0 | 1971.5 | 232.1 |
| DIPE g/hr/l cat. | 48.4 | 33.1 | 29.2 | 24.4 | 33.8 | 18.5 | 0.0 | 0.0 |
| IPA/IPA+HC+DIPE | 0.425 | 0.611 | 0.565 | 0.392 | 0.505 | 0.750 | 0.000 | 0.545 |

5

As these data show, water strongly inhibits polymerisation. For a pure propylene feed, hydrocarbon production was 14 tines that for an equimolar water:propylene feed (Runs 7 and 8). It is unnecessary to employ a large excess of water (e.g., 10:1 mole ratios as shown in US-A-4,499,313, supra) as the latter does not exert a proportional suppression of hydrocarbon coproduction but has the disadvantage of requiring greater water recycle capability.

EXAMPLE 3

This example demonstrates the superior activity of zeolite Beta (hydrogen form, binder free) compared to typical acid ion exchange resin catalysts for the hydration of propylene under fairly severe process conditions including high water to propylene mole ratios. The zeolite Beta possesses a silica to alumina ratio of 41.

The conditions of hydration and the results thereof are set forth in Table 3 as follows:

Table 3 : Comparison of Zeolite Beta with Acid Ion Exchange Catalysts at High Water: Propylene Mole Ratios

| Hydration Conditions | Zeolite Beta | Ion Exchange Resin Amberlite 250 | Amberlyst 15 |
|---|---|---|---|
| Temp, °C | 150 | 150 | 149 |
| Pressure, psig (bar) | 1000 (70) | 1200 (83.8) | 1000 |
| Water:Propylene Mole Ratio | 10 | 14 | 7 |
| % Propylene Conversion | 49 | 45 | 36 |
| IPA/IPA+DIPE+HC | 0.9 | ~1 | ~1 |
| SPACE TIME YIELDS | | | |
| g-IPA/L-cat hr | 188 | 108 | 105 |
| g-DIPE/L-cat hr | 19 | 4 | 2 |
| g-HC/L-cat hr | – | – | – |
| g-Total/L-cat hr | 207 | 112 | 107 |

As these data show, under relatively severe conditions, zeolite Beta is nearly twice as active as the acid ion exchange resins.

EXAMPLE 4

Three grams of unbound zeolite Beta (in the hydrogen form) having a silica to alumina ratio of 40 and an alpha value of 424 were placed in a stainless steel reactor. The reactor was purged with helium and brought to 150°C and 1000 psig (70 bar). Propylene and water were separately fed into the reactor. The weight hourly space velocity of liquid propylene was maintained at 0.5 and the mole ratio of water:propylene in the feed was varied from 10:1 to 1:1. Gas and liquid products were collected and analysed chromatographically. The results are set forth in Table 4 as follows:

6

Table 4:    Propylene_Hydration_Over_Zeolite_Beta

| Water:Propylene Mole Ratio | 10/1 | 5/1 | 2/1 | 1/1 |
|---|---|---|---|---|
| % Propylene Conversion | 49 | 40 | 37 | 30 |
| Product Selectivity, wt% | | | | |
|    IPA | 91 | 79 | 59 | 57 |
|    DIPE | 9 | 20 | 39 | 41 |
|    Others | 0 | 1 | 2 | 2 |

As the data in Table 4 show, once-through propylene conversion of 49% (63% of equilibrium conversion) was obtained with 100% selectivity to IPA and DIPE (91% and 9% by weight, respectively). The results are striking in terms of activity, selectivity, and the quantity of DIPE produced at such a high ratio of water:propylene. DIPE selectivity increased sharply with decreasing water:propylene ratio (from 9% at 10:1 to 41 wt% at 1:1 ratio).

EXAMPLE 5
- - - - - - -

This example demonstrates the superior performance of zeolite Beta catalyst bound with 35 weight percent alumina compared to several other known olefin hydration catalysts when used in the hydration of propylene at relatively high temperatures. The reaction conditions and results are set forth in Table 5 as follows:

Table 5:    High Temperature Propylene Hydration Processes

| Hydration Conditions | Zeolite Beta | $SiO_2/Al_2O_3$ (Solid) | W/Al (Solid) | $Na_3HSW^*$ (Liquid) | $H_3PO_4$ (Supported Liquid) | 60% $H_3PO_4$ (Supported Liquid) |
|---|---|---|---|---|---|---|
| Temp., °C | 206 | 270 | 270 | 260 | 218 | 171 |
| Pressure, psig (bar) | 1400 (97.6) | 3700 (254.2) | 3700 | 3000 (207.9) | 590 (41.7) | 428 (30.5) |
| Water:Propylene Mole Ratio | 1 | 15 | 16 | 28 | 1 | 0.24 |
| % Propylene Conversion | 17 | 45 | 18 | 64 | 6 | 8 |
| IPA/IPA+DIPE+HC | 0.9 | 1 | 1 | 1 | 1 | 1 |
| SPACE TIME YIELDS g-IPA/L-cat hr | 1660 | 522 | 1020 | 260 | 103 | 630 |

\* $Na_3H[Si(W_3O_{10})_4]$ in water, 0.001 M

The comparison indicates that zeolite Beta showed higher IPA space time yield than the other catalysts, particularly those operating at comparable or lower conversion. In particular, zeolite Beta showed 60% higher space time yield than the tungsten catalyst operating at the same conversion but at twice the pressure and 60°C higher temperature. The space time yield for zeolite Beta was much higher than the remaining catalysts (HSW, $SiO_2/Al_2O_3$) although the latter were operated at higher temperatures, pressure and water co-feed.

EXAMPLE 6
- - - - - - -

The performance of zeolite Beta (unbound) for the hydration of propylene at a water:propylene mole ratio of 10:1, compared with other large pore zeolites is set forth in Table 6 as follows:

7

Table 6 : Comparison of Propylene Hydration With Zeolite Beta and Other Large Pore Zeolites

| Zeolite | Zeolite Beta | UltraStable Zeolite Y | Zeolite Y | Mordenite |
|---|---|---|---|---|
| SIO$_2$/Al$_2$O$_3$ | 40 | 10 | 150 | 64 |
| Propylene Conversion, % | 49 | <1 | 16 | 44 |
| Product Selectivity, wt% | | | | |
| IPA | 91 | 100 | 98 | 91 |
| DIPE | 9 | 0 | 2 | 6 |
| Propylene Oligomers | 0 | 0 | 0 | 3 |

As these data show, the percentage of conversion of propylene was significantly higher under the stated conditions than the next most active large pore zeolite, mordenite; and, unlike the latter, no appreciable amounts of propylene oligomers were present in the mixture of product IPA and DIPE.

EXAMPLE 7

The performance of unbound zeolite Beta for the hydration of propylene at water:propylene mole ratios of 2:1 compared with other large pore zeolites at 1000 psig (70 bar), 330°F (166°C) and 0.6 WHSV propylene is set forth in Table 7 as follows:

Table 7: Comparison of Propylene Hydration With Zeolite Beta and Other Large Pore Zeolites at Water:Propylene Mole Ratios of 2:1

| Zeolite | SiO$_2$/Al$_2$O$_3$ | Alpha | Propylene Conversion, % 2:1 H$_2$O:C$_3$H$_6$ | IPA 2:1 H$_2$O:C$_3$H$_6$ | Selectivities DIPE 2:1 H$_2$O:C$_3$H$_6$ | Oligomers 2:1 H$_2$O:C$_3$H$_6$ |
|---|---|---|---|---|---|---|
| Beta | 35 | 289 | 51.0 | 48.7 | 48.2 | 3.1 |
| Mordenite | 25 | 62 | 5.7 | 96.5 | 3.5 | 0.0 |
| USY | 5 | 144 | 1.8 | 100.0 | 0.0 | 0.0 |

As these data show, when the mole ratio of olefin and propylene are reduced to well under the 10:1 level of Example 6, the advantages of zeolite Beta over the other large pore zeolites becomes more pronounced. Thus, propylene conversion where zeolite Beta is employed is much higher and selectivity to oxygenates is maintained.

EXAMPLE 8

This example compares the performance of zeolite Beta for the hydration of propylene at a water:propylene mole ratio of 10:1 with that of a medium pore zeolite, ZSM-5. The other conditions of the hydrations were: 330°F(166°C), 1000 psig (70 bar) and 0.5 WHSV propylene. The results are set forth in Table 8 as follows:

Table 8: Comparison of Propylene Hydration
With Zeolite Beta and ZSM-5 at 10:1
Propylene:Water Mole Ratio

| Zeolite | Zeolite Beta | ZSM-5 |
|---|---|---|
| $SiO_2/Al_2O_3$ | 40 | 70 |
| Alpha | 424 | 310 |
| Propylene Conversion, % | 49 | 22 |
| Product Selectivity, % | | |
| IPA | 91 | 98 |
| DIPE | 9 | 2 |
| Propylene Oligomers | 0 | 0 |

As these data show, the percentage of propylene conversion was dramatically higher in the case of zeolite Beta and, in addition, substantially more DIPE was produced employing the zeolite Beta catalyst.

EXAMPLE 9

This example compares the results obtained with unbound zeolite Beta and zeolite Beta bound with varying amounts of two different binders, silica and alumina. The alpha values of the different catalysts are set forth in Table 9 as follows:

Table 9: Alpha Values of Unbound and Bound
Zeolite Beta

| Catalyst | Composition (Wt%) | Alpha Value (of the Un-bound zeolite) | Unsteamed or Steamed |
|---|---|---|---|
| A | Unbound Zeolite Beta | 438 | Unsteamed |
| B | 65 Zeolite Beta/ 35% $SiO_2$ | 359 | Unsteamed |
| C | 65% Zeolite Beta/ 35% $Al_2O_3$ | 281 | Unsteamed |

The hydration runs were conducted in a 3/8" (0.95 cm) stainless steel reactor at 1000 psig (70 bar) in a downflow operation (propylene in gas phase and water in liquid phase). In each run, 5 grams of each of Catalysts A, B and C (based on zeolite component) (12-20 mesh) (1.68-0.84 mm) were loaded into the reactor and the unit was pressurised with helium to 1000 psig. Propylene and water were fed separately into the top of the reactor at a rate ten times that of the desired space velocity. The reactor was then gradually brought up to the desired operating temperature at which point the rates of introduction of propylene and water were reduced to the desired space velocities. The temperature profile was quite uniform (±1.5°F (0.83°C) variation). After lineout, gas and liquid samples were collected periodically (generally over a period of 17 hours for each sample) and were analysed by gas chromatography. The results of the hydration runs are set forth in Table 10 as follows:

Table 10: Propylene Hydration Over Unbound and Bound Zeolite Beta

|  | Catalyst A | | | Catalyst B | | | Catalyst C | | |
|---|---|---|---|---|---|---|---|---|---|
| Temp., C | 150 | 150 | | 150 | 150 | | 150 | 150 | |
| Mole Ratio of Water:Propylene | 1:1 | 1:1 | | 1:1 | 1:1 | | 1:1 | 1:1 | |
| Propylene WHSV (based on catalyst) | .72 | .72 | | .5 | .5 | | .5 | .5 | |
| Propylene WHSV (based on zeolite) | .72 | .72 | | .77 | .77 | | .77 | .77 | |
| Time on Stream, hrs. | 5-25 | 29-42 | | 5-22 | 29-52 | | 6-24 | 30-48 | |
| Propylene Conversion, % | 38.2 | 38.0 | Avg. 38.1 | 30.6 | 31.5 | Avg. 31.1 | 15.2 | 14.5 | Avg. 14.9 |
| Product Distribution, wt% | | | | | | | | | |
| IPA | 40.9 | 42.1 | 41.5 | 45.3 | 44.5 | 44.9 | 55.0 | 59.6 | 57.3 |
| DIPE | 57.3 | 56.2 | 56.7 | 53.1 | 54.0 | 53.5 | 43.5 | 38.6 | 41.0 |
| Propylene Oligomers | 1.8 | 1.7 | 1.8 | 1.6 | 1.5 | 1.6 | 1.5 | 1.8 | 1.7 |

EXAMPLE 10

This example explores the relationship between catalyst acidity (expressed in terms of alpha value) and catalyst performance. At 302°F (150°C), 1000 psig (70 bar), 0.77 propylene WHSV (based on zeolite) and 1:1 mole ratio of water: propylene, using Catalyst B (alpha value of 359), Catalyst D (steamed 65% zeolite Beta/35% $SiO_2$, alpha value of 272) and Catalyst E (steamed 65% zeolite Beta/35% $SiO_2$, alpha value of 35), propylene conversions of 31%, 26% and 12%, respectively, were obtained. A favourable relationship between increased alpha values and percentage of propylene conversion were also noted with alumina-bound zeolite Beta. Thus, under substantially similar olefin hydration conditions, Catalyst C (alpha value of 281) provided 15% propylene conversion whereas Catalyst F (steamed 65% zeolite Beta/35 wt% $Al_2O_3$) provided only 7% conversion.

EXAMPLE 11

Unsteamed, unbound zeolite Beta (Catalyst 1) was evaluated for the hydration of propylene under the conditions, and with the results, set forth in Table 11 as follows:

Table 11: Effect of Process Variables on Performance of Catalyst A

| Temp., °F (°C) | 302 (150) | 324 (162) |
|---|---|---|
| Pressure, psig (bar) | 1000 (70) | 1000 |
| Mole Ratio Water/Propylene | 1:1 | 1:1 |
| Propylene WHSV (based on zeolite) | 0.5 | 0.5 |
| Propylene Conversion, % | 49.2 | 61.8 |
| Product Distribution, wt% | | |
| IPA | 40.5 | 42.9 |
| DIPE | 57.6 | 54.8 |
| Propylene Oligomers | 1.9 | 2.3 |

10

As these data show, at a mole ratio of water:propylene of 1:1 and a propylene WHSV of 0.5, an increase in temperature of from 302ºF to 324ºF increased the percentage propylene conversion from 49 to 62%. However, an increase in the temperature to 324ºF resulted in a small decrease in propylene conversion possibly resulting from unfavourable thermodynamic equilibrium at the higher temperature.

**Claims**

1. A process for converting light olefins to alcohols and/or ethers which comprises contacting a feed containing at least one light olefin with water in the vapour and/or liquid phase at a water to olefin mole ratio of at least 1 under olefin hydration conditions in the presence of a catalyst comprising zeolite Beta.

2. A process according to claim 1 wherein the feed contains a mixture of light olefins.

3. A process according to claim 1 or claim 2 wherein the olefins contain from two to seven carbon atoms.

4. A process according to any preceding claim wherein the feed contains ethylene, propylene, a butene, a pentene, a hexane and/or a heptene.

5. A process according to any preceding claim wherein the feed comprises naphtha cracker off-gas.

6. A process according to any preceding claim wherein the feed is catalytically cracked light gasoline containing pentenes, hexenes and heptenes.

7. A process according to any preceding claim wherein the conditions include a temperature of 50º to 300ºC.

8. A process according to claim 7 wherein the temperature is 50º to 220ºC.

9. A process according to claim 8 wherein the temperature is 90º to 200ºC.

10. A process according to any preceding claim wherein the conditions include a total system pressure of at least 5 atm (bar).

11. A process according to claim 10 wherein the total system pressure is at least 20 atm (bar).

12. A process according to claim 11 wherein the total system pressure is at least 40 atm (bar).

13. A process according to any preceding claim wherein the mole ratio of water to total olefin is up to 30.

14. A process according to claim 13 wherein the mole ratio of water to total olefin is up to 15.

15. A process according to claim 14 wherein the mole ratio of water to total olefin is up to 5.

16. A process according to any preceding claim wherein the zeolite Beta is composited with a binder.

17. A process according to claim 16 wherein the binder comprises silica and/or alumina.

18. A process according to any preceding claim wherein the catalyst is an extrudate containing an extrusion facilitating amount of a low acidity refractory oxide binder in the colloidal state which is substantially free of added alkali metal base or basic salt.

19. A process according to any preceding claim wherein the alpha value of the zeolite Beta is at least 1.

20. A process according to any preceding claim wherein the alpha value of the zeolite Beta is at least 10.

21. A process according to any preceding claim wherein the alpha value of the zeolite Beta is at least 100.

**22.** A process according to any preceding claim wherein the zeolite is in the hydrogen form.

**23.** A process according to any preceding claim wherein the catalyst further comprises a co-catalyst effective for the hydration of light olefin.

**Patentansprüche**

**1.** Verfahren zur Umwandlung leichter Olefine zu Alkoholen und/oder Ethern, das den Kontakt einer Beschickung, die mindestens ein leichtes Olefin enthält, mit Wasser in der Dampf- und/oder Flüssigkeitsphase bei einem Molverhältnis von Wasser/Olefin von mindestens 1 und bei Olefinhydratationsbedingungen in Gegenwart eines Katalysators umfaßt, der Zeolith Beta umfaßt.

**2.** Verfahren nach Anspruch 1, worin die Beschickung eine Mischung aus leichten Olefinen enthält.

**3.** Verfahren nach Anspruch 1 oder 2, worin die Olefine von 2 bis 7 Kohlenstoffatome enthalten.

**4.** Verfahren nach einem der vorstehenden Ansprüche, worin die Beschickung Ethylen, Propylen, Buten, Penten, Hexan und/oder Hepten enthalten.

**5.** Verfahren nach einem der vorstehenden Ansprüche, worin die Beschickung das Abgas der Crackvorrichtung für Rohbenzin umfaßt.

**6.** Verfahren nach einem der vorstehenden Ansprüche, worin die Beschickung katalytisch gecracktes geringsiedendes Benzin ist, das Pentene, Hexene und Heptene enthält.

**7.** Verfahren nach einem der vorstehenden Ansprüche, worin die Bedingungen eine Temperatur von 50 bis 300°C umfassen.

**8.** Verfahren nach Anspruch 7, worin die Temperatur 50 bis 220°C beträgt.

**9.** Verfahren nach Anspruch 8, worin die Temperatur 90 bis 200°C beträgt.

**10.** Verfahren nach einem der vorstehenden Ansprüche, worin die Bedingungen einen Gesamtdruck des Systems von mindestens 5 atm (bar) umfassen.

**11.** Verfahren nach Anspruch 10, worin der Gesamtdruck des Systems mindestens 20 atm (bar) beträgt.

**12.** Verfahren nach Anspruch 11, worin der Gesamtdruck des Systems mindestens 40 atm (bar) beträgt.

**13.** Verfahren nach einem der vorstehenden Ansprüche, worin das Molverhältnis von Wasser zum gesamten Olefin bis zu 30 beträgt.

**14.** Verfahren nach Anspruch 13, worin das Molverhältnis von Wasser zum gesamten Olefin bis zu 15 beträgt.

**15.** Verfahren nach Anspruch 14, worin das Molverhältnis von Wasser zum gesamten Olefin bis zu 5 beträgt.

**16.** Verfahren nach einem der vorstehenden Ansprüche, worin der Zeolith Beta mit einem Bindemittel zusammengesetzt ist.

**17.** Verfahren nach Anspruch 16, worin das Bindemittel Siliciumdioxid und/oder Aluminiumoxid umfaßt.

**18.** Verfahren nach einem der vorstehenden Ansprüche, worin der Katalysator ein Extrudat ist, das eine die Extrusion erleichternde Menge eines hitzebeständigen Oxidbindemittels mit geringer Acidität im kolloidalen Zustand enthält, die im wesentlichen frei von einer zugesetzten Alkalimetallbase oder einem basischen Salz ist.

**19.** Verfahren nach einem der vorstehenden Ansprüche, worin der $\alpha$-Wert des Zeolith Beta mindestens 1 beträgt.

**20.** Verfahren nach einem der vorstehenden Ansprüche, worin der $\alpha$-Wert des Zeolith Beta mindestens 10 beträgt.

**21.** Verfahren nach einem der vorstehenden Ansprüche, worin der $\alpha$-Wert des Zeolith Beta mindestens 100 beträgt.

**22.** Verfahren nach einem der vorstehenden Ansprüche, worin der Zeolith in der Wasserstofform vorliegt.

**23.** Verfahren nach einem der vorstehenden Ansprüche, worin der Katalysator außerdem einen Katalysatorpartner umfaßt, der für die Hydratation des leichten Olefins effektiv ist.

**Revendications**

**1.** Un procédé pour convertir des oléfines légères en alcools et/ou éthers, qui consiste à mettre en contact une charge contenant au moins une oléfine légère avec de l'eau en phase vapeur et/ou en phase liquide, pour un rapport en moles de l'eau à l'oléfine au moins égal à 1, dans les conditions d'une hydratation des oléfines, en présence d'un catalyseur comprenant une zéolite béta.

**2.** Un procédé selon la revendication 1, dans lequel la charge contient un mélange d'oléfines légères.

**3.** Un procédé selon la revendication 1 ou la revendication 2, dans lequel les oléfines ont de 2 à 7 atomes de carbone.

**4.** Un procédé selon l'une quelconque des revendications précédentes, dans lequel la charge contient de l'éthylène, du propylène, un butène, un pentène, un hexène et/ou un heptène.

**5.** Un procédé selon l'une quelconque des revendications précédentes, dans lequel la charge comprend un gaz d'échappement d'une unité de craquage de naphta.

**6.** Un procédé selon l'une quelconque des revendications précédentes, dans lequel la charge est une essence légère ayant subi un craquage catalytique et contenant des pentènes, des hexènes et des heptènes.

**7.** Un procédé selon l'une quelconque des revendications précédentes, dans lequel les conditions comprennent une température de 50 à 300°C.

**8.** Un procédé selon la revendication 7, dans lequel la température est de 50 à 220°C.

**9.** Un procédé selon la revendication 8, dans lequel la température est de 90 à 200°C.

**10.** Un procédé selon l'une quelconque des revendications précédentes, dans lequel les conditions comprennent une pression totale du système d'au moins 5 atm. (bar).

**11.** Un procédé selon la revendication 10, dans lequel la pression totale du système est d'au moins 20 atm. (bar).

**12.** Un procédé selon la revendication 11, dans lequel la pression totale du système est d'au moins 40 atm. (bar).

**13.** Un procédé selon l'une quelconque des revendications précédentes, dans lequel le rapport en moles de l'eau à l'oléfine totale est inférieur ou égal à 30.

**14.** Un procédé selon la revendication 13, dans lequel le rapport en moles de l'eau à l'oléfine totale est inférieur ou égal à 15.

**15.** Un procédé selon la revendication 14, dans lequel le rapport en moles de l'eau à l'oléfine totale est inférieur ou égal à 5.

**16.** Un procédé selon l'une quelconque des revendications précédentes, dans lequel la zéolite béta est mélangée à un liant.

**17.** Un procédé selon la revendication 16, dans lequel le liant comprend de la silice et/ou de l'alumine.

**18.** Un procédé selon l'une quelconque des revendications précédentes, dans lequel le catalyseur est un extrudat contenant une quantité, facilitant l'extrusion, d'un liant oxyde réfractaire à faible acidité, à l'état colloïdal, et auquel il n'a pratiquement pas été ajouté d'hydroxyde de métal alcalin ou d'un sel basique d'un métal alcalin.

**19.** Un procédé selon l'une quelconque des revendications précédentes, dans lequel l'indice alpha de la zéolite béta est au moins égal à 1.

**20.** Un procédé selon l'une quelconque des revendications précédentes, dans lequel l'indice alpha de la zéolite béta est au moins égal à 10.

**21.** Un procédé selon l'une quelconque des revendications précédentes, dans lequel l'indice alpha de la zéolite béta est au moins égal à 100.

**22.** Un procédé selon l'une quelconque des revendications précédentes, dans lequel la zéolite est sous forme protonée.

**23.** Un procédé selon l'une quelconque des revendications précédentes, dans lequel le catalyseur comprend en outre un cocatalyseur assurant l'hydratation des oléfines légères.